# EUROPEAN PATENT APPLICATION

(11) **EP 4 477 170 A1**
(43) Date of publication of application: **18.12.2024**
(21) Application number: 23763383.9
(22) Date of filing: 27.02.2023
(51) Int. Cl.: A61B 18/14

(54) **MEDICAL DEVICE**

(30) Priority: 01.03.2022 JP 2022030844
(71) Applicant: Terumo Kabushiki Kaisha, Tokyo 151-0072 (JP)
(72) Inventor: TAKAHASHI, Yusuke, Ashigarakami-gun, Kanagawa 259-0151 (JP)
(74) Representative: Prüfer & Partner mbB Patentanwälte · Rechtsanwälte
(86) International application number: PCT/JP2023/006959
(87) International publication number: WO 2023/167125

(57) **Abstract**

Provided is a medical device with which force applied in a longitudinal direction to a flexible printed wiring board when a shaft section is bent can be reduced and with which pliability in the direction of bending of the shaft section can also be improved. The present invention is a medical device 10 provided with: an expanding body 21, a shaft section 20, an energy transmission section that runs along the expanding body 21, and a conducting section 60 that supplies power to the energy transmission section. The conducting section 60 comprises an integrated planar member that includes: a deforming section 65 that deforms in accordance with expansion and contraction of the expanding body 21; and an extension section 66 that extends from a base end of the deforming section 65 in a base-end direction. In the deforming section 65, a width direction of the planar member is roughly parallel to a direction of a tangent to a circle centered on a central axis of the expanding body 21. The extension section 66 comprises: a bending section 68 where a direction of extension of the extension section 66 in a longitudinal direction bends from a direction of extension of the deforming section 65 in a longitudinal direction; and a helical section 69 that extends in the base-end direction from the bending section 68 and is wound in a helical shape onto an inner tube 20a.

## Description

### Technical Field

The present invention relates to a medical device that applies energy to a biological tissue.

### Background Art

A medical device is known that includes an electrode portion arranged in an expansion body that expands and contracts in a living body, and performs a treatment by ablation to cauterize a biological tissue by a high-frequency current from the electrode portion. As one of treatments by ablation, a shunt treatment on the atrial septum is known. The shunt treatment can alleviate heart failure symptoms of a patient with heart failure by forming a shunt (puncture hole) serving as an escape route for an increased atrial pressure in the fossa ovalis of the atrial septum of the patient. In the shunt treatment, the atrial septum is accessed using an intravenous approaching method, and a shunt with a desired size is formed.

The electrode portion of the expansion body is electrically connected to an energy applying device disposed on a hand side and can receive energy supply. Such a medical device is disclosed in, for example, Patent Literature 1.

### Citation List

### Patent Literature

Patent Literature 1: WO 2019-085841

### Summary of Invention

### Technical Problem

The electrode portion of the expansion body is disposed on the flexible printed wiring board. The flexible printed wiring board extends from the expansion body to the base end side along the shaft portion. The flexible printed wiring board has an elongated flat plate shape. Therefore, when the flexible printed wiring board is arranged along the shaft portion, a force of stretching or compressing the flexible printed wiring board is applied when the shaft portion is bent, which might cause breakage. Since the flexible printed wiring board has low elasticity in a length direction, flexibility for bending the shaft portion is reduced.

The present invention has been made to solve the above-described problem, and an object thereof is to provide a medical device configured to reduce a force applied to a flexible printed wiring board in a length direction when a shaft portion is bent and improving flexibility of the shaft portion in a bending direction.

### Solution to Problem

A medical device according to the present invention to achieve the above-described object includes an expansion body that includes a central axis and can expand and contract in a radial direction, an elongated shaft portion that includes a connection portion to the expansion body on a distal end, the shaft portion that includes an inner tube and an outer tube covering the inner tube, an energy transmission portion disposed along the expansion body in an intermediate portion of the expansion body in a direction of the central axis, and a conductive portion for supplying power to the energy transmission portion, in which the conductive portion is formed of an integrated flat plate-shaped member including a base surface extending in a width direction and a length direction, including a deformation portion extending from the energy transmission portion to the distal end of the shaft portion and deforming according to expansion and contraction of the expansion body, and an extension portion extending from a proximal end of the deformation portion in a proximal direction and arranged between the inner tube and the outer tube, the deformation portion is arranged in such a manner that a width direction of the flat plate-shaped member in the deformation portion is substantially parallel to a tangential direction with respect to a circle centered on the central axis of the expansion body, and the extension portion includes a bent portion in which a direction extending in a length direction of the extension portion is bent from a direction extending in a length direction of the deformation portion, and a spiral portion extending in a proximal direction from the bent portion and spirally wound around the inner tube in a state in which the base surface of the flat plate-shaped member faces an outer peripheral surface of the inner tube.

### Advantageous Effects of Invention

In the medical device configured in the above-described manner, since the spiral portion is spirally wound around the shaft portion, it is possible to reduce a force applied to the conductive portion in the length direction when the shaft portion is bent, and to easily bend the shaft portion. Since the structure for connecting the conductive portion to a power supply unit can be disposed on a side proximal of the connection portion between the expansion body and the shaft portion so as not to overlap each other, flexibility on the distal end of the shaft portion can be secured. Since the deformation portion along the expansion body is formed of a flat plate-shaped member, it is possible to deform following the expansion and contraction of the expansion body without twisting.

The energy transmission portion and the conductive portion may be formed of an integrated flexible printed wiring board including a conductive layer and an insulating layer surrounding an outer surface of the conductive layer, and the energy transmission portion may be an electrode portion disposed in such a manner that a part of the conductive layer is exposed from the insulating layer. This can reduce a stretching or compressing force on the flexible printed wiring board.

The extension portion may include the linear portion extending substantially in the direction of the central axis of the shaft portion between the proximal end of the deformation portion and the bent portion. As a result, the deformation portion and the bent portion are separated from each other, so that it is possible to prevent the deformation of the deformation portion from being transmitted to the bent portion to damage the bent portion.

The energy transmission portion may include a plurality of transmission body portions arranged at substantially regular intervals in a circumferential direction centered on a central axis of the expansion body, the deformation portion may include a plurality of deformation body portions extending from the plurality of transmission body portions, respectively, and the linear portion of the extension portion may include a plurality of linear body portions extending from the plurality of deformation body portions, respectively. As a result, the plurality of conductive portions can be easily arranged at substantially regular intervals in the circumferential direction of the expansion body.

The shaft portion may include a guide portion in which the plurality of linear body portions is arranged at substantially regular intervals in the circumferential direction centered on the central axis of the expansion body in the vicinity of the connection portion. As a result, the plurality of conductive portions can be reliably arranged at substantially regular intervals in the circumferential direction.

The bent portion may include a plurality of bent body portions connected to proximal ends of the plurality of deformed body portions, respectively, and the spiral portion may include a plurality of spiral body portions extending from proximal ends of the plurality of bent body portions, respectively. This can simplify the structure of the conductive portion.

The extension portion may include the joining portion that connects proximal ends of at least two of the plurality of linear body portions, and the bent portion may be connected to the proximal end of the joining portion. As a result, the number of spiral portions wound around the shaft portion can be reduced, and the spiral portion can be easily wound.

The plurality of transmission body portions may include a plurality of first electrode portions and a plurality of second electrode portions that form a pair, the deformation portion may include a plurality of first deformation body portions extending from the plurality of first electrode portions, respectively, and a plurality of second deformation body portions extending from the plurality of second electrode portions, respectively, the extension portion may include a first joining portion connecting proximal ends of the plurality of first deformation body portions and a second joining portion connecting proximal ends of the plurality of second deformation body portions, the bent portion may include a first bent portion connected to a proximal end of the first joining portion and a second bent portion connected to a proximal end of the second joining portion, and the spiral portion may include a first spiral portion extending from a proximal end of the first bent portion and a second spiral portion extending from a proximal end of the second bent portion. As a result, in a case where the transmission main body is a bipolar electrode, the number of spiral portions can be reduced to facilitate winding around the shaft portion.

The shaft portion may include a curved portion shaped to bend at a certain angle in advance in a portion where the spiral portion is spirally wound around the inner tube. As a result, when arranging the expansion body in the atrial septum, it becomes easy to adjust a direction of the expansion body to the atrial septum, and the living tissue can be reliably gripped by the expansion body.

### Brief Description of Drawings

Fig. 1 is a front view illustrating an overall configuration of a medical device according to an embodiment.
Fig. 2 is an enlarged perspective view of the vicinity of an expansion body.
Fig. 3 is an enlarged front view of the vicinity of the expansion body.
Fig. 4 is a cross-sectional view of a flexible printed wiring board.
Fig. 5 is an arrangement diagram illustrating an arrangement relationship of a plurality of flexible printed wiring boards in a circumferential direction in a vertical direction of a paper surface.
Fig. 6 is a perspective view of a distal end of a shaft portion in a state in which the shaft portion is cut in a position proximal of the expansion body.
Fig. 7 is a cross-sectional view of a medical device taken along a cross section orthogonal to an axial direction on a proximal end of the expansion body.
Fig. 8 is an enlarged front view of a portion of the shaft portion adjacent to a proximal end side of the expansion body.
Fig. 9 is an enlarged front view of the vicinity of a distal end of the medical device.
Fig. 10 is an illustrative diagram for schematically illustrating a state in which the expansion body is arranged in an atrial septum, including a front view of the medical device and a cross-sectional view of a biological tissue.
Fig. 11 is an enlarged front view illustrating a state in which the expansion body grips a living tissue.
Fig. 12 is an arrangement diagram illustrating an arrangement relationship of a plurality of flexible printed wiring boards according to a first modification in a vertical direction of a paper surface.
Fig. 13 is an arrangement diagram illustrating an arrangement relationship of a plurality of flexible printed wiring boards according to a second modification in a vertical direction of a paper surface.
Fig. 14 is an enlarged front view of the vicinity of an expansion body of a medical device according to a modification.
Fig. 15 is an enlarged front view of the vicinity of an expansion body of a medical device according to a second modification.

### Description of Embodiments

Hereinafter, an embodiment of the present invention is described with reference to the drawings. Note that, dimensional ratios in the drawings are sometimes exaggerated and different from actual ratios for convenience of description. In this specification, a side inserted into a biological lumen of a medical device 10 is referred to as a "distal end" or a "distal end side", and an operating hand side is referred to as a "proximal end" or a "proximal end side".

The medical device according to the embodiment described below is configured to expand a puncture hole Hh formed in an atrial septum HA of a heart H of a patient, and to further perform a maintenance treatment to maintain the expanded puncture hole Hh at that size.

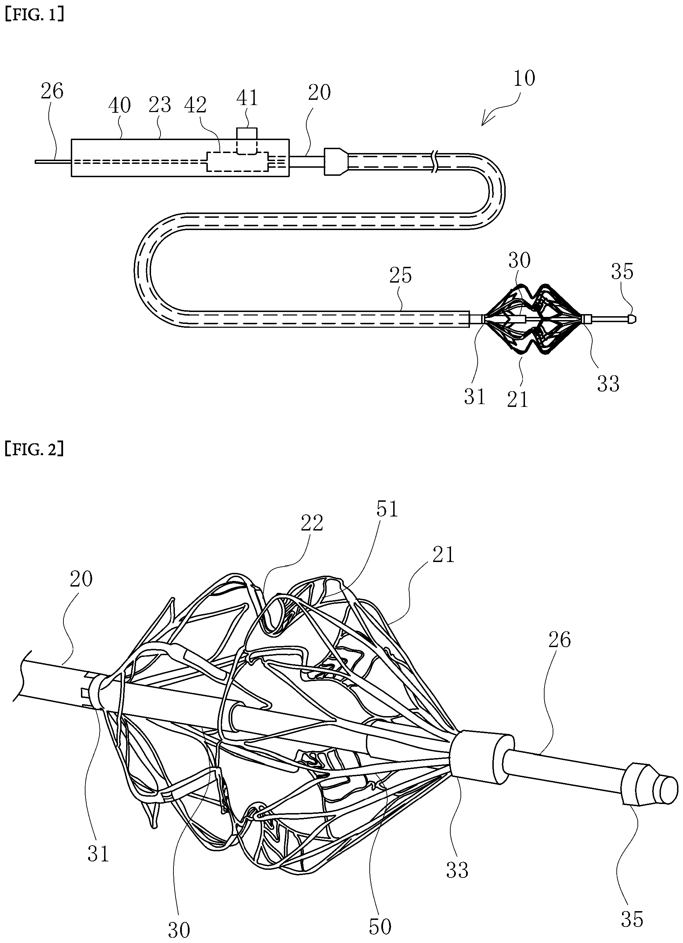

As illustrated in Fig. 1, the medical device 10 according to this embodiment includes an elongated shaft portion 20, an expansion body 21 disposed on a distal end of the shaft portion 20, and a hand operation unit 23 disposed on a proximal end of the shaft portion 20. On the expansion body 21, an electrode portion 22, which is an energy transmission element for performing the above-described maintenance treatment, is disposed.

The shaft portion 20 includes a connection portion 31 to which a proximal end of the expansion body 21 is fixed, and a first shaft portion 30 extending from the connection portion 31 into the expansion body 21. The first shaft portion 30 extends along a central axis of the expansion body 21 from the vicinity of the proximal end of the expansion body 21 to the middle of the expansion body 21, specifically, to the vicinity of a recess 51 of the expansion body 21 to be described later.

The shaft portion 20 includes a storage sheath 25 disposed on an outermost peripheral portion. The expansion body 21 is movable forward and rearward in an axial direction with respect to the storage sheath 25. The storage sheath 25 can house the expansion body 21 therein in a state of moving to the distal end side of the shaft portion 20. The expansion body 21 can be exposed by moving the storage sheath 25 from a state of housing the expansion body 21 to the proximal end side.

A pulling shaft 26 is arranged in the shaft portion 20 so as to be slidable with respect to the shaft portion 20. The pulling shaft 26 is disposed from a position proximal of the hand operation unit 23 to a position distal of the expansion body 21. The pulling shaft 26 protrudes from the distal end of the shaft portion 20, specifically, from the first shaft portion 30, passes through the inside of the expansion body 21, further passes through a distal end fixing portion 33 to which a distal end of the expansion body 21 is fixed, and protrudes from the distal end of the expansion body 21. A distal end of the pulling shaft 26 is fixed to a distal end member 35.

The distal end member 35 to which the distal end of the pulling shaft 26 is fixed does not need to be fixed to the expansion body 21. As a result, when the pulling shaft 26 slides in a proximal direction with respect to the shaft portion 20, the distal end member 35 can apply a compressing force to the expansion body 21 along an axial center of the shaft portion 20. When the expansion body 21 is stored in the storage sheath 25, by separating the distal end member 35 toward the distal end side from the expansion body 21, the expansion body 21 can be easily moved in an extending direction, so that storability is improved.

The hand operation unit 23 includes a housing 40 gripped by an operator, an operation dial 41 that can be rotationally operated by the operator, and a conversion mechanism 42 operated in conjunction with the rotation of the operation dial 41. The pulling shaft 26 is held by the conversion mechanism 42 inside the hand operation unit 23. The conversion mechanism 42 can move the pulling shaft 26 held by the same forward and rearward in the axial direction in conjunction with the rotation of the operation dial 41. For example, a rack and pinion mechanism can be used as the conversion mechanism 42.

It is preferable that the shaft portion 20 be formed of a material having a certain degree of flexibility. Examples of such a material include, for example, polyolefin such as polyethylene, polypropylene, polybutene, ethylene-propylene copolymer, ethylene-vinyl acetate copolymer, ionomer, or mixture of two or more of them, soft polyvinyl chloride resin, polyamide, polyamide elastomer, polyester, polyester elastomer, polyurethane, fluorine resin such as polytetrafluoroethylene, polyimide, PEEK, silicone rubber, latex rubber and the like.

The pulling shaft 26 can be formed of, for example, an elongated wire material such as a super elastic alloy such as a nickel-titanium alloy and a copper-zinc alloy, a metal material such as stainless steel, and a resin material having comparatively high rigidity.

The distal end member 35 can be formed of, for example, a super elastic alloy such as a nickel-titanium alloy or a copper-zinc alloy, a metal material such as stainless steel, a polymer material such as polyolefin, polyvinyl chloride, polyamide, polyamide elastomer, polyurethane, polyurethane elastomer, polyimide, and fluororesin, or a mixture thereof, a multilayer tube of two or more kinds of polymer materials or the like.

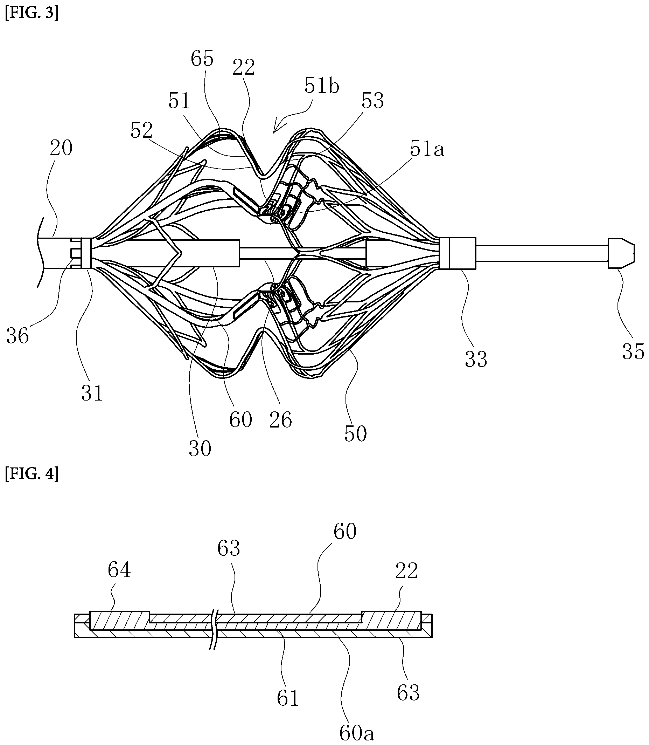

As illustrated in Figs. 2 and 3, the expansion body 21 includes a plurality of wire material portions 50 in a circumferential direction. The wire material portions 50 form a mesh-shaped structure by branching and joining in a length direction. As a result, the expansion body 21 can expand and contract in a radial direction. A proximal end of the wire material portion 50 extends from the connection portion 31 to the distal end side. A distal end of the wire material portion 50 extends from a proximal end of the distal end fixing portion 33 to the proximal end side. The wire material portion 50 is inclined to be larger in the radial direction from both ends toward a central portion in the axial direction. The wire material portion 50 includes the recess 51 in the central portion in the axial direction, the recess 51 recessed radially inward of the expansion body 21. An innermost part of the recess 51 in the radial direction is a bottom portion 51a. The recess 51 defines a reception space 51b configured to receive a biological tissue when the expansion body 21 expands.

The recess 51 includes a proximal end side upright portion 52 extending radially outward from a proximal end of the bottom portion 51a and a distal end side upright portion 53 extending radially outward from a distal end of the bottom portion 51a. When the pulling shaft 26 slides in the proximal direction with respect to the shaft portion 20 and the compressing force is applied to the expansion body 21, the distal end side upright portion 53 and the proximal end side upright portion 52 approach each other, and both portions come in close contact with the biological tissue received in the reception space 51b. On the proximal end side upright portion 52, the electrode portion 22 is arranged along the recess 51 so as to face the reception space 51b. That is, the electrode portion 22 is disposed along the expansion body 21 in an intermediate portion in a central axis direction of the expansion body 21. In this embodiment, six electrode portions 22 are disposed in the circumferential direction. Note that, the electrode portion 22 may be arranged on the distal end side upright portion 53.

The wire material portions 50 forming the expansion body 21 can be formed by cutting a single metal cylindrical member with a laser or the like. The wire material portion 50 can be formed of a metal material. As the metal material, a titanium-based alloy (Ti-Ni, Ti-Pd, Ti-Nb-Sn or the like), a copper-based alloy, stainless steel, β-titanium steel, and a Co-Cr alloy may be used, for example. Note that, an alloy having a spring property such as a nickel-titanium alloy can be more preferably used. Note that, the material of the wire material portion 50 is not limited to them, and other materials may be used.

A distal end shaft portion including the connection portion 31 of the shaft portion 20, the first shaft portion 30, the distal end fixing portion 33, and a part of the pulling shaft 26 exposed to the inside of the expansion body 21 between the first shaft portion 30 and the distal end fixing portion 33 is arranged inside the expansion body 21. The distal end shaft extends from the proximal end to the distal end of the expansion body 21. As a result, when the expansion body 21 expands, the central axis of the expansion body 21 is prevented from being excessively bent.

The electrode portion 22 is disposed on a conductive portion 60 and is connected to an energy supply device (not illustrated), which is an external device, via the conductive portion 60. A high-frequency voltage is applied from the energy supply device to an electrode pair including two electrode portions 22 via the conductive portion 60, and energy is applied between them. That is, the electrode portion 22 is formed as a bipolar electrode.

The conductive portion 60 is arranged in the length direction of the wire material portion 50. The conductive portion 60 is a flexible printed wiring board formed of a flat plate-shaped member including a base surface 60a elongated in the length direction and extending in the width direction and the length direction. Note that, the flexible printed wiring board described herein does not form an electrical circuit by itself, and the electrical circuit is formed when this is connected to the energy supply device.

As illustrated in Fig. 4, the conductive portion 60 has a multilayer structure including a conductive layer 61, an insulating layer 63 surrounding an outer surface of the conductive layer 61, and an adhesive layer 62 disposed between the conductive layer 61 and the insulating layer 63. The electrode portion 22, which is an energy transmission portion, is disposed in such a manner that a part of the conductive layer 61 is exposed from the insulating layer 63. A contact portion 64 for connection to a conductive wire is disposed on a proximal end of the conductive portion 60. The contact portion 64 is disposed in such a manner that a part of the conductive layer 61 is exposed from the insulating layer 63.

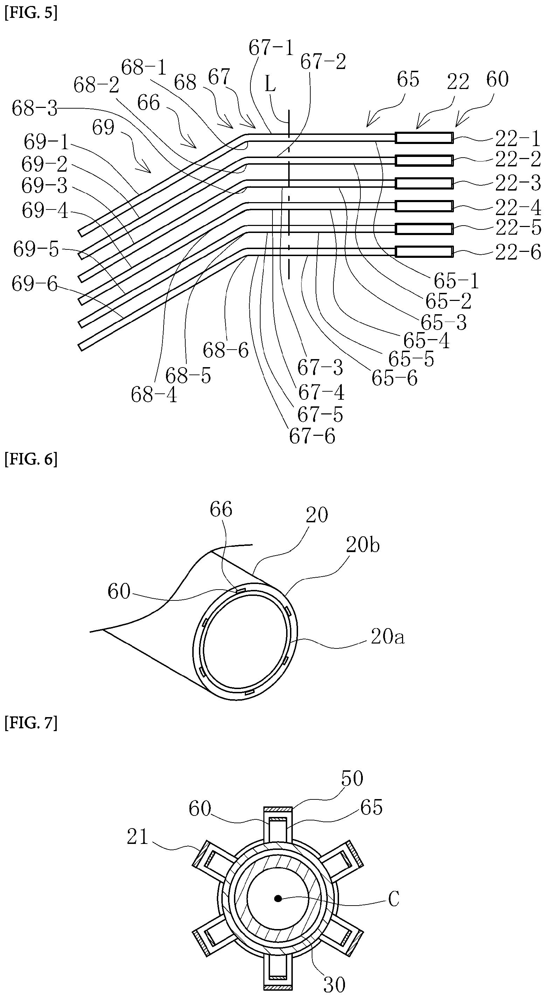

As illustrated in Fig. 5, in the conductive portion 60, a deformation portion 65 extending from the electrode portion 22 to a position of the connection portion 31 of the shaft portion 20 indicated by a broken line L, and an extension portion 66 extending from a proximal end of the deformation portion in the proximal direction are integrally formed.

As illustrated in Fig. 6, the shaft portion 20 includes an inner tube 20a including a lumen and an outer tube 20b covering an outer side in a radial direction of the inner tube 20a. The extension portion 66 of the conductive portion 60 is arranged between the inner tube 20a and the outer tube 20b in the shaft portion 20 and extends in an axial direction thereof. Note that, the outer tube 20b is not illustrated in subsequent drawings so that the conductive portion 60 is displayed.

As illustrated in Fig. 7, the deformation portion 65 is arranged in such a manner that the width direction of the conductive portion 60 in the deformation portion 65 is substantially parallel to a tangential direction with respect to a circle centered on a central axis C of the expansion body 21. Therefore, the deformation portion 65 extends along the wire material portion 50 of the expansion body 21, and can be deformed without being twisted according to expansion and contraction of the expansion body 21.

As illustrated in Fig. 5, the extension portion 66 includes a linear portion 67 extending from the proximal end of the deformation portion 65 in the proximal direction, a bent portion 68 located on a proximal end side of the linear portion 67 in which a direction extending in a length direction of the extension portion 66 is bent from a direction extending in a length direction of the deformation portion 65, and a spiral portion 69 extending from the bent portion 68 in the proximal direction.

Six conductive portions 60 each including the electrode portion 22 are disposed in the circumferential direction of the shaft portion 20. The electrode portion 22 includes a plurality of transmission body portions 22-1, 22-2, 22-3, 22-4, 22-5, and 22-6 arranged at substantially regular intervals in the circumferential direction centered on the central axis of the expansion body 21. In the transmission body portions, positive electrodes and negative electrodes are alternately arranged in the circumferential direction. The deformation portion 65 includes deformation body portions 65-1, 65-2, 65-3, 65-4, 65-5, and 65-6 extending from a plurality of transmission body portions, respectively. The linear portion 67 includes linear body portions 67-1, 67-2, 67-3, 67-4, 67-5, and 67-6 extending from a plurality of deformation body portions, respectively. The bent portion 68 includes bent body portions 68-1, 68-2, 68-3, 68-4, 68-5, and 68-6 connected to the proximal ends of a plurality of deformation body portions, respectively. The spiral portion 69 includes spiral body portions 69-1, 69-2, 69-3, 69-4, 69-5, and 69-6 extending from proximal ends of a plurality of bent body portions, respectively.

The bent body portions 68-1, 68-2, 68-3, 68-4, 68-5, and 68-6 are arranged in the same position in the length direction of the shaft portion 20, and are all bent at the same angle. The bending angle of the bent body portions 68-1, 68-2, 68-3, 68-4, 68-5, and 68-6 is 30°. Note that, the bending angle is not limited thereto, and can be set to any angle in a range of 15° to 45°. When the positions of the bent body portions 68-1, 68-2, 68-3, 68-4, 68-5, and 68-6 are the same in the length direction of the shaft portion 20 and the bending angle is within this range, it is possible to prevent the spiral body portions 69-1, 69-2, 69-3, 69-4, 69-5, and 69-6 from interfering with one another.

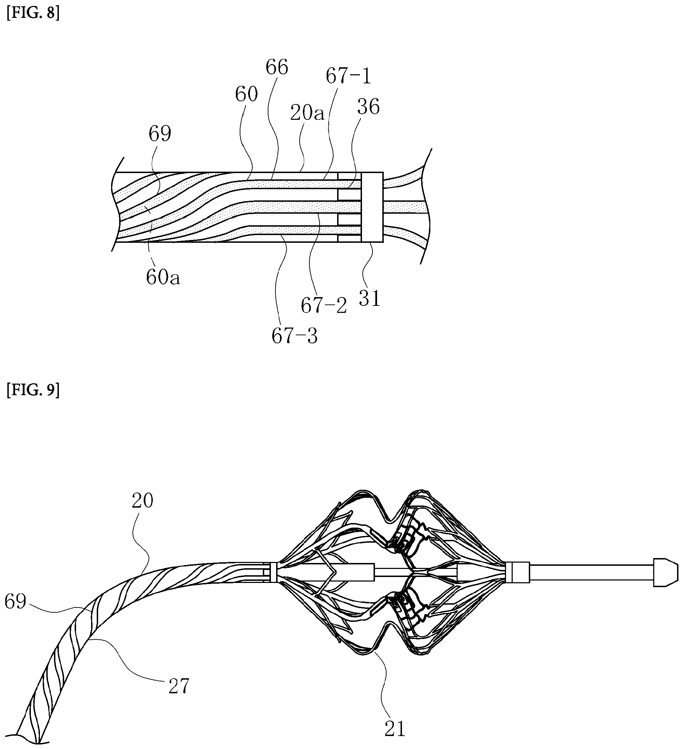

As illustrated in Fig. 8, the shaft portion 20 includes a guide portion 36 in which the linear body portions 67-1, 67-2, and 67-3 are arranged at substantially regular intervals in the circumferential direction centered on the central axis of the expansion body 21 in the vicinity of the connection portion 31. The extension portions 66 of the conductive portion 60 are arranged at substantially regular intervals in the circumferential direction by the guide portion 36.

The spiral portion 69 extending in the proximal direction from the bent portion 68 is spirally wound around the inner tube 20a in a state in which the base surface 60a faces an outer peripheral surface of the inner tube 20a. When manufacturing the medical device 10, the spiral portion 69 is wound around the inner tube 20a in a linear state as illustrated in Fig. 8, and by bending a portion of the shaft portion 20 around which the spiral portion 69 is wound as illustrated in Fig. 9, a curved portion 27 is formed in the shaft portion 20. Since the shaft portion 20 includes the curved portion 27, it becomes easy to adjust the expansion body 21 to the angle of the atrial septum, and the living tissue can be reliably gripped by the expansion body 21.

In the conductive portion 60, since the spiral portion 69 is spirally wound around the shaft portion 20, a force applied in the length direction is reduced even when the shaft portion 20 is bent, and a risk of breakage can be reduced. Since the bending of the shaft portion 20 is not hindered by the conductive portion 60, the flexibility of the shaft portion 20 in the bending direction can be improved.

In the conductive portion 60, a proximal end of the spiral portion 69 is connected to the conductive wire, and the conductive wire is connected to a connector portion (not illustrated) of the energy supply device. Three conductive wires having the same polarity of the electrode portions 22 join together in the connector portion. A joining position is not limited thereto, and may be joined at the conducting wire.

A method using the medical device 10 is described. The medical device 10 is used in a treatment to be performed on a patient suffering from chronic heart failure in which myocardial hypertrophy appears in a left ventricle of the heart H and stiffness (hardness) increases so that a blood pressure increases in a left atrium HLa.

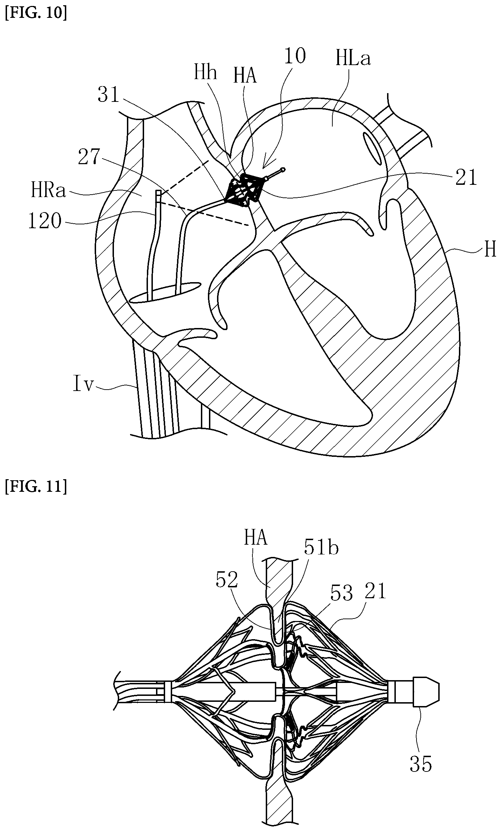

As illustrated in Fig. 10, the medical device 10 is delivered from an inferior vena cava Iv to the vicinity of the atrial septum HA via a right atrium HRa, and is arranged in the position of the puncture hole Hh in which the expansion body 21 is formed in advance. A distal end of the medical device 10 penetrates the atrial septum HA and reaches the left atrium HLa. When inserting the medical device 10, the expansion body 21 is housed in the storage sheath 25, and the expansion body 21 is exposed by moving the storage sheath 25 to the proximal end side. As a result, the expansion body 21 increases in diameter, and the recess 51 is arranged in the puncture hole Hh of the atrial septum HA and receives the biological tissue surrounding the puncture hole Hh in the reception space 51b. The shaft portion 20 includes the curved portion 27 as described above, and the curved portion 27 can be exposed from the storage sheath 25 to be curved in one direction. This allows the expansion body 21 to be arranged in such a manner that the axial direction thereof is close to perpendicular to a plane of the atrial septum HA. Note that, the medical device 10 may be a device without the storage sheath 25. In this case, a sheath corresponding to the storage sheath 25 is separately prepared, and the sheath is delivered to the vicinity of the atrial septum HA along the guide wire 11 in advance so that a distal end of the sheath reaches the left atrium HLa via the through-hole Hh of the atrial septum HA. Next, the expansion body 21 of the medical device 10 is inserted into the sheath from a proximal end of the sheath, and the distal end of the expansion body 21 is delivered to the left atrium HLa via the through-hole Hh of the atrial septum HA in a similar manner to Fig. 10.

As illustrated in Fig. 11, by moving the pulling shaft 26 to the proximal end side in a state in which the reception space 51b receives the biological tissue, the expansion body 21 is pulled in a compression direction by the distal end member 35 to be compressed in the axial direction, the atrial septum HA is gripped by the proximal end side upright portion 52 and the distal end side upright portion 53, and the electrode portion 22 is pressed against the biological tissue.

By applying high-frequency energy to an edge of the puncture hole Hh via the electrode portion 22 in a state in which the electrode portion 22 is pressed against the biological tissue, the edge of the puncture hole Hh can be cauterized (heated and cauterized) with the high-frequency energy. The high-frequency energy is applied by applying a voltage between a pair of electrode portions 22 adjacent in the circumferential direction. As a result, it is possible to inhibit blockage due to natural healing of the puncture hole Hh and maintain the size thereof.

When using the medical device 10, hemodynamics is checked by a hemodynamics checking device 120 delivered to the right atrium HRa via the inferior vena cava Iv. As the hemodynamics checking device 120, a known echo catheter can be used, for example. The operator can display an echo image acquired by the hemodynamics checking device 120 on a display device such as a display, and can check an amount of blood passing through the puncture hole Hh on the basis of a displayed result.

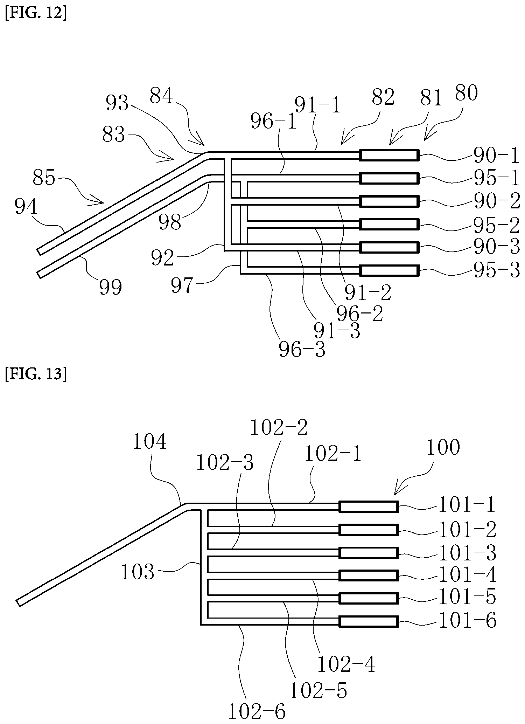

Next, a modification of the conductive portion is described. As illustrated in Fig. 12, a conductive portion 80 includes an electrode portion 81, a deformation portion 82, and an extension portion 83. The extension portion 83 includes a bent portion 84 and a spiral portion 85. The electrode portion 81 includes a plurality of first electrode portions 90-1, 90-2, and 90-3, and a plurality of second electrode portions 95-1, 95-2, and 95-3 forming a pair. The first electrode portions 90-1, 90-2, and 90-3, and the second electrode portions 95-1, 95-2, and 95-3 are arranged so as to be adjacent to each other in the circumferential direction of the expansion body 21. The deformation portion 82 includes first deformation body portions 91-1, 91-2, and 91-3 extending from the first electrode portions 90-1, 90-2, and 90-3, respectively, and second deformation body portions 96-1, 96-2, and 96-3 extending from the second electrode portions 95-1, 95-2, and 95-3, respectively. The extension portion 83 includes a first joining portion 92 connecting proximal ends of the first deformation body portions 91-1, 91-2, and 91-3 and a second joining portion 97 connecting proximal ends of the second deformation body portions 96-1, 96-2, and 96-3. The bent portion 84 includes a first bent portion 93 connected to a proximal end of the first joining portion 92 and a second bent portion 98 connected to a proximal end of the second joining portion 97. The spiral portion 85 includes a first spiral portion 94 extending from a proximal end of the first bent portion 93 and a second spiral portion 99 extending from a proximal end of the second bent portion 98. In this manner, the spiral portions may be integrated for each polarity of the electrode portion 81, and the number of wires spirally wound around the shaft portion 20 can be reduced.

The energy transmission portion may be a monopolar electrode. In this case, energization is performed with an external electrode. As illustrated in Fig. 13, a conductive portion 100 includes electrode portions 101-1, 101-2, 101-3, 101-4, 101-5, and 101-6 all having the same polarity, and six linear body portions 102-1, 102-2, 102-3, 102-4, 102-5, and 102-6 have their proximal ends joined to form a joining portion 103. The bent portion 104 is connected to a proximal end of the joining portion 103. In this manner, all the conductive portions 100 may be integrated by the joining portion 103. The number of conductive portions 100 joined by the joining portion 103 may be any number of two or more.

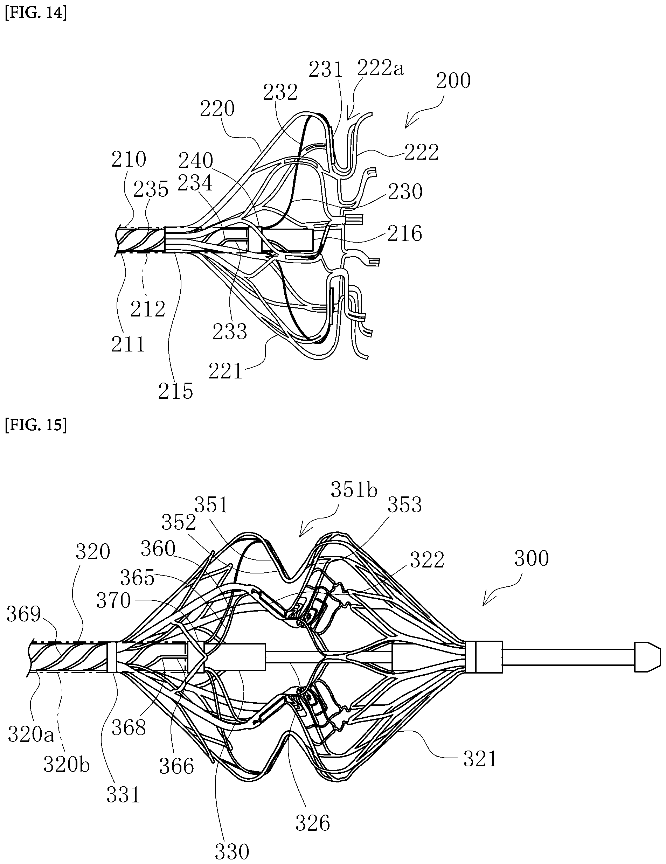

A medical device 200 according to a modification is described. As illustrated in Fig. 14, the medical device 200 according to the modification includes an expansion body 220 on a distal end of a shaft portion 210. The expansion body 220 includes an enlarged diameter portion 221 that expands from the distal end of the shaft portion 210 toward a distal end side and a recess 222 in which an electrode portion 231 is arranged, and the recess 222 is a most distal end. The expansion body 220 may be expanded by a self-expanding force. In this example, the pulling shaft is not disposed, and the expansion body 220 self-expands, so that a width in an axial direction of a receiving space 222a formed by the recess 222 is narrowed, and the living tissue can be gripped.

The shaft portion 210 includes a connection portion 215 to which a proximal end of the expansion body 220 is fixed, and a first shaft portion 216 extending from the connection portion 215 into the expansion body 220. An inner tube 211 extends to a distal end of the first shaft portion 216. An outer tube 212 indicated by a broken line in the drawing includes a distal end located inside the expansion body 220 and is located closer to the connection portion 215 than a distal end of the inner tube 211. On a side distal of the outer tube 212 of the inner tube 211, a convergence tube 240 that bundles and holds a plurality of conductive portions 230 is disposed. The convergence tube 240 may be formed of, for example, a heat-shrink tube. Note that, the convergence tube 240 is not necessarily disposed.

The conductive portion 230 includes a deformation portion 232 extending from the electrode portion 231 arranged in the expansion body 220 to the convergence tube 240 arranged on the distal end of the shaft portion 210, and an extension portion 233 extending in the proximal direction from a proximal end of the deformation portion 232 and arranged between the inner tube 211 and the outer tube 212. The extension portion 233 includes a spiral portion 235 that is bent at a bent portion 234, extends in the proximal direction from the bent portion 234, and is wound around the inner tube 211 in a spiral shape.

In this manner, the extension portion 233 of the conductive portion 230 may be arranged inside the expansion body 220. The medical device 200 of this example can be assembled by attaching the expansion body 220 to an assembly of the inner tube 211, the outer tube 212, and the conductive portion 230 created in advance. Therefore, efficiency of assembly workability can be improved.

Next, a medical device 300 according to a second modification is described. As illustrated in Fig. 15, the medical device 300 according to the second modification includes an expansion body 321 on a distal end of a shaft portion 320. A configuration of the expansion body 321 is similar to that of the expansion body 21 in Fig. 3, and a receiving space 351b is formed of a recess 351 including a proximal end side upright portion 352 and a distal end side upright portion 353. A width in an axial direction of the receiving space 351b can be narrowed by a pulling shaft 326 to grip the biological tissue.

The shaft portion 320 includes a connection portion 331 to which a proximal end of the expansion body 320 is fixed, and a first shaft portion 330 extending from the connection portion 331 into the expansion body 321. An inner tube 320a extends to a distal end of the first shaft portion 330. An outer tube 320b indicated by a broken line in the drawing includes a distal end located inside the expansion body 321 and is located closer to the connection portion 331 than a distal end of the inner tube 320a. On part of the inner tube 320a distal of the outer tube 320b, a convergence tube 370 that bundles and holds a plurality of conductive portions 360 is disposed. Note that, the convergence tube 370 is not necessarily disposed.

The conductive portion 360 includes a deformation portion 365 extending from the electrode portion 322 arranged in the expansion body 321 to the convergence tube 370 arranged on the distal end of the shaft portion 320, and an extension portion 366 extending in the proximal direction from a proximal end of the deformation portion 365 and arranged between the inner tube 320a and the outer tube 320b. The extension portion 366 includes a spiral portion 369 that is bent at a bent portion 368, extends in the proximal direction from the bent portion 368, and is wound around the inner tube 320a in a spiral shape. In this manner, the extension portion 366 of the conductive portion 360 may be arranged inside the expansion body 321 also in the expansion body 321 similar to that in Fig. 3.

As described above, a medical device 10 according to this embodiment includes an expansion body 21 that includes a central axis and can expand and contract in a radial direction, an elongated shaft portion 20 that includes a connection portion 31 to the expansion body 21 on a distal end, the shaft portion 20 that includes an inner tube 20a and an outer tube 20b covering the inner tube 20a, an energy transmission portion disposed along the expansion body 21 in an intermediate portion of the expansion body 21 in a direction of the central axis, and a conductive portion 60 for supplying power to the energy transmission portion, in which the conductive portion 60 is formed of an integrated flat plate-shaped member including a base surface 60a extending in a width direction and a length direction, including a deformation portion 65 extending from the energy transmission portion to the distal end of the shaft portion 20 and deforming according to expansion and contraction of the expansion body 21, and an extension portion 66 extending from a proximal end of the deformation portion 65 in a proximal direction and arranged between the inner tube 20a and the outer tube 20b, the deformation portion 65 is arranged in such a manner that a width direction of the flat plate-shaped member in the deformation portion 65 is substantially parallel to a tangential direction with respect to a circle centered on the central axis of the expansion body 21, and the extension portion 66 includes a bent portion 68 in which a direction extending in a length direction of the extension portion 66 is bent from a direction extending in a length direction of the deformation portion 65, and a spiral portion 69 extending in a proximal direction from the bent portion 68 and spirally wound around the inner tube 20a in a state in which the base surface 60a of the flat plate-shaped member faces an outer peripheral surface of the inner tube 20a. In the medical device 10 configured in this manner, since the spiral portion 69 is spirally wound around the shaft portion 20, it is possible to reduce a force applied to the conductive portion 60 in the length direction when the shaft portion 20 is bent, and to easily bend the shaft portion 20. Since the structure for connecting the conductive portion 60 to a power supply unit can be disposed on a side proximal of the connection portion 31 between the expansion body 21 and the shaft portion 20 so as not to overlap each other, flexibility on the distal end of the shaft portion 20 can be secured. Since the deformation portion 65 along the expansion body 21 is formed of a flat plate-shaped member, it is possible to deform following the expansion and contraction of the expansion body 21 without twisting.

The energy transmission portion and the conductive portion 60 may be formed of an integrated flexible printed wiring board including the conductive layer 61 and the insulating layer 63 surrounding an outer surface of the conductive layer 61, and the energy transmission portion may be the electrode portion 22 disposed in such a manner that a part of the conductive layer 61 is exposed from the insulating layer 63. This can reduce a stretching or compressing force on the flexible printed wiring board.

The extension portion 66 may include the linear portion 67 extending substantially in the direction of the central axis of the shaft portion 20 between the proximal end of the deformation portion 65 and the bent portion 68. As a result, the deformation portion 65 and the bent portion 68 are separated from each other, so that it is possible to prevent the deformation of the deformation portion 65 from being transmitted to the bent portion 68 to damage the bent portion 68.

The energy transmission portion may include a plurality of transmission body portions 22-1, 22-2, 22-3, 22-4, 22-5, and 22-6 arranged at substantially regular intervals in the circumferential direction centered on the central axis of the expansion body 21, the deformation portion 65 may include a plurality of deformation body portions 65-1, 65-2, 65-3, 65-4, 65-5, and 65-6 extending from a plurality of transmission body portions, respectively, and the linear portion 67 of the extension portion 66 may include a plurality of linear body portions 67-1, 67-2, 67-3, 67-4, 67-5, and 67-6 extending from the plurality of deformation body portions, respectively. As a result, the plurality of conductive portions 60 can be easily arranged at substantially regular intervals in the circumferential direction of the expansion body 21.

The bent portion 68 may include a plurality of bent body portions 68-1, 68-2, 68-3, 68-4, 68-5, and 68-6 connected to proximal ends of the plurality of deformation body portions, and the spiral portion 69 may include a plurality of spiral body portions 69-1, 69-2, 69-3, 69-4, 69-5, and 69-6 extending from proximal ends of the plurality of bent body portions, respectively. This can simplify the structure of the conductive portion 60.

The shaft portion 20 may include the guide portion 36 that arranges the plurality of linear body portions 67-1, 67-2, 67-3, 67-4, 67-5, and 67-6 at substantially regular intervals in the circumferential direction centered on the central axis of the expansion body 21 in the vicinity of the connection portion 31. As a result, the extension portions 66 of the conductive portion 60 are reliably arranged at substantially regular intervals in the circumferential direction.

The extension portion may include the joining portion 103 that connects proximal ends of at least two of the plurality of linear body portions 102-1, 102-2, 102-3, 102-4, 102-5, and 102-6, and the bent portion 104 may be connected to the proximal end of the joining portion 103. As a result, the number of spiral portions wound around the shaft portion 20 can be reduced, and the spiral portion can be easily wound.

The plurality of transmission body portions may include a plurality of first electrode portions 90 and a plurality of second electrode portions 95 that form a pair, the deformation portion 82 may include a plurality of first deformation body portions 91-1, 91-2, and 91-3 extending from the plurality of first electrode portions 90, respectively, and a plurality of second deformation body portions 96-1, 96-2, and 96-3 extending from the plurality of second electrode portions 95, respectively, the extension portion 83 may include a first joining portion 92 connecting proximal ends of the plurality of first deformation body portions and a second joining portion 97 connecting proximal ends of the plurality of second deformation body portions, the bent portion 84 may include a first bent portion 93 connected to a proximal end of the first joining portion 92 and a second bent portion 98 connected to a proximal end of the second joining portion 97, and the spiral portion 85 may include a first spiral portion 94 extending from a proximal end of the first bent portion 93 and a second spiral portion 99 extending from a proximal end of the second bent portion 98. As a result, in a case where the transmission main body is a bipolar electrode, the number of spiral portions can be reduced to facilitate winding around the shaft portion 20.

The shaft portion 20 may include a curved portion shaped to bend at a certain angle in advance in a portion where the spiral portion 69 is spirally wound around the inner tube 20a. As a result, when arranging the expansion body 21 in the atrial septum, it becomes easy to adjust a direction of the expansion body 21 to the atrial septum, and the living tissue can be reliably gripped by the expansion body 21.

Note that, the present invention is not limited to the embodiment described above, and various modifications may be made by those skilled in the art within the technical idea of the present invention.

Note that, the present application is based on Japanese Patent Application No. 2022-30844 filed on March 1, 2022, the entire disclosed content of which is incorporated herein by reference.

### Reference Signs List

- 10: Medical device
- 11: Guide wire
- 20: Shaft portion
- 20a: Inner tube
- 20b: Outer tube
- 21: Expansion body
- 22: Electrode portion (energy transmission portion)
- 27: Curved portion
- 31: Connection portion
- 36: Guide portion
- 50: Wire material portion
- 51: Recess
- 60: Conductive portion
- 60a: Base surface
- 61: Conductive layer
- 62: Adhesive layer
- 63: Insulating layer
- 64: Contact portion
- 65: Deformation portion
- 66: Extension portion
- 67: Linear portion
- 68: Bent portion
- 69: Spiral portion
- 70: Transmission body portion
- 71: Deformation body portion
- 72: Extension body portion
- 73: Linear body portion
- 74: Bent body portion
- 75: Spiral body portion

## Claims

1. A medical device comprising:
an expansion body that includes a central axis and can expand and contract in a radial direction;
an elongated shaft portion that includes a connection portion to the expansion body on a distal end, the shaft portion that includes an inner tube and an outer tube covering the inner tube;
an energy transmission portion disposed along the expansion body in an intermediate portion of the expansion body in a direction of the central axis; and
a conductive portion for supplying power to the energy transmission portion, wherein
the conductive portion is formed of an integrated flat plate-shaped member including a base surface extending in a width direction and a length direction, including a deformation portion extending from the energy transmission portion to the distal end of the shaft portion and deforming according to expansion and contraction of the expansion body, and an extension portion extending from a proximal end of the deformation portion in a proximal direction and arranged between the inner tube and the outer tube,
the deformation portion is arranged in such a manner that a width direction of the flat plate-shaped member in the deformation portion is substantially parallel to a tangential direction with respect to a circle centered on the central axis of the expansion body, and
the extension portion includes a bent portion in which a direction extending in a length direction of the extension portion is bent from a direction extending in a length direction of the deformation portion, and a spiral portion extending in a proximal direction from the bent portion and spirally wound around the inner tube in a state in which the base surface of the flat plate-shaped member faces an outer peripheral surface of the inner tube.

2. The medical device according to claim 1, wherein the energy transmission portion and the conductive portion are formed of an integrated flexible printed wiring board including a conductive layer and an insulating layer surrounding an outer surface of the conductive layer, and
the energy transmission portion is an electrode portion disposed in such a manner that a part of the conductive layer is exposed from the insulating layer.

3. The medical device according to claim 1 or 2, wherein the extension portion includes a linear portion extending substantially in a direction of the central axis of the shaft portion between a proximal end of the deformation portion and the bent portion.

4. The medical device according to claim 3, wherein the energy transmission portion includes a plurality of transmission body portions arranged at substantially regular intervals in a circumferential direction centered on a central axis of the expansion body,
the deformation portion includes a plurality of deformation body portions extending from the plurality of transmission body portions, respectively, and
the linear portion of the extension portion includes a plurality of linear body portions extending from the plurality of deformation body portions, respectively.

5. The medical device according to claim 4, wherein the shaft portion includes a guide portion in which the plurality of linear body portions is arranged at substantially regular intervals in the circumferential direction centered on the central axis of the expansion body in the vicinity of the connection portion.

6. The medical device according to claim 4 or 5, wherein the bent portion includes a plurality of bent body portions connected to proximal ends of the plurality of deformed body portions, respectively, and
the spiral portion includes a plurality of spiral body portions extending from proximal ends of the plurality of bent body portions, respectively.

7. The medical device according to claim 4 or 5, wherein the extension portion includes a joining portion that connects proximal ends of at least two of the plurality of linear body portions, and
the bent portion is connected to a proximal end of the joining portion.

8. The medical device according to claim 4 or 5, wherein the plurality of transmission body portions includes a plurality of first electrode portions and a plurality of second electrode portions that form a pair,
the deformation portion includes a plurality of first deformation body portions extending from the plurality of first electrode portions, respectively, and a plurality of second deformation body portions extending from the plurality of second electrode portions, respectively,
the extension portion includes a first joining portion connecting proximal ends of the plurality of first deformation body portions and a second joining portion connecting proximal ends of the plurality of second deformation body portions,
the bent portion includes a first bent portion connected to a proximal end of the first joining portion and a second bent portion connected to a proximal end of the second joining portion, and
the spiral portion includes a first spiral portion extending from a proximal end of the first bent portion and a second spiral portion extending from a proximal end of the second bent portion.

9. The medical device according to any one of claims 1 to 8, wherein the shaft portion includes a curved portion shaped to bend at a certain angle in advance in a portion in which the spiral portion is spirally wound around the inner tube.
